# EUROPÄISCHE PATENTSCHRIFT

(11) **EP 3 175 240 B1**
(45) Veröffentlichungstag und Bekanntmachung des Hinweises auf die Patenterteilung: **22.08.2018**
(21) Anmeldenummer: 15747420.6
(22) Anmeldetag: 30.07.2015
(51) Int. Cl.: G01N 33/543

(54) **VERFAHREN ZUR BINDUNG VON BIOLOGISCH AKTIVEN MOLEKÜLEN AN OBERFLÄCHEN**
METHODS FOR BINDING BIOLOGICALLY ACTIVE MOLECULES TO SURFACES
PROCÉDÉ DE LIAISON DE MOLÉCULES BIOLOGIQUEMENT ACTIVES À DES SURFACES

(30) Priorität: 01.08.2014 DE 102014215208
(43) Veröffentlichungstag der Anmeldung: 07.06.2017
(73) Patentinhaber: Soskic, Vukic, 55131 Mainz (DE); Poppe, Robert, 55128 Mainz (DE)
(72) Erfinder: Soskic, Vukic, 55131 Mainz (DE); Poppe, Robert, 55128 Mainz (DE)
(74) Vertreter: Weickmann & Weickmann PartmbB
(86) Internationale Anmeldenummer: PCT/EP2015/067523
(87) Internationale Veröffentlichungsnummer: WO 2016/016377

(56) Entgegenhaltungen:
- SUN XUE-LONG ET AL: "Carbohydrate and protein immobilization onto solid surfaces by sequential Diels-Alder and azide-alkyne cycloadditions", BIOCONJUGATE CHEMISTRY, ACS, WASHINGTON, DC, US, Bd. 17, Nr. 1, 18. Januar 2006 (2006-01-18), Seiten 52-57, XP002588543, ISSN: 1043-1802, DOI: 10.1021/BC0502311 [gefunden am 2005-12-21]
- PRIM DENIS ET AL: "ADIBO-based "click" chemistry for diagnostic peptide micro-array fabrication: physicochemical and assay characteristics.", MOLECULES (BASEL, SWITZERLAND) 2013, Bd. 18, Nr. 8, 2013, Seiten 9833-9849, XP9186176, ISSN: 1420-3049

## Beschreibung

### Gebiet der Erfindung

Die vorliegende Erfindung betrifft Verfahren zur adsorptiven Bindung von biologisch aktiven Molekülen auf Oberflächen indem die Oberfläche in einem ersten Schritt mit einem Trägerreagenz beschichtet wird und die zu bindenden biologisch aktiven Moleküle anschließend durch eine biorthogonale Cycloadditionsreaktion kovalent an das Trägerreagenz gekoppelt werden. Anwendungsgebiete der Erfindung sind die biochemische Forschung, die medizinische Diagnostik und die pharmazeutische Industrie.

### Hintergrund

Zahlreiche medizinische und pharmazeutische Bindungsassays basieren auf dem Prinzip eines Festphasenimmunoassays. Hierbei wird ein biologisch aktives Molekül, häufig ein Protein, an einer festen Trägeroberfläche wie z.B. an Kunststoffpartikel oder an der Innenwand von Reaktionsgefäßen wie den Kavitäten einer Mikrotiterplatte gebunden. Üblicherweise erfolgt die Bindung hierbei durch Adsorption an der Oberfläche. Diese direkte Adsorption ist ein sehr einfaches Bindungsverfahren, dessen Effizienz aber für unterschiedliche zu beschichtende Moleküle sehr variabel ist. Zusätzlich werden Proteine bei der Adsorption an einer hydrophoben Oberfläche leicht denaturiert und verlieren so an biologischer Aktivität. Bei niedermolekularen Substanzen besteht hingegen das Problem, dass diese durch einfache Adsorption an der Oberfläche nur unzureichend gebunden werden. Zusätzlich besteht das Problem, dass insbesondere niedermolekulare Substanzen bei direkter Beschichtung auf einer Oberfläche nicht mehr mit einem zu analysierenden Bindungspartner interagieren können. Um diese Einschränkungen zu umgehen werden Moleküle häufig in Biotin-modifizierter Form auf Oberflächen aufgebracht die mit einem Biotinbindeprotein wie z.B. Streptavidin vorbeschichtet sind. Aber auch dieses Verfahren ist mit zahlreichen Einschränkungen behaftet. Im Streptavidinmolekül ist die Bindekapazität für biotinylierte Moleküle auf maximal 4 Valenzen pro Streptavidintetramer eingeschränkt, wodurch die Beladungsdichte der Oberfläche reduziert wird. Weiterhin wird Streptavidin durch die i.d.R. passive hydrophobe Adsorption an der Oberfläche teilweise denaturiert sowie sterisch eingeschränkt so dass die Bindekapazität für biotinmodifizierte Moleküle weiter reduziert wird. Darüber hinaus sind mit Avidin- oder Streptavidin vorbeschichtete Oberflächen anfällig für unspezifische Interferenzreaktionen, z.B. durch immunologische Kreuzreaktionen mit Streptavidin oder durch die Bindung biotinhaltiger Moleküle aus der zu analysierenden Probe.

US 5,279,955 (Pegg et al.) beschreibt heterofunktionale quervernetzende Reagenzien die über eine hydrophobe Kohlenwasserstoffkette an Plastikoberflächen adsorbieren und über eine quervernetzende Hydroxysuccinimidfunktion die zu beschichtenden Moleküle kovalent binden. Dieses Verfahren ist jedoch mit dem Nachteil behaftet, dass sich das niedermolekulare Trägerreagenz durch detergenzhaltige Puffer von der Plastikoberfläche ablösen kann.

US 6,713,272 (Anderson et al.) beschreibt ein Verfahren zur Bindung von Biomolekülen an hydrophobe Oberflächen auf der Basis eines rekombinanten Proteins das mit einer Lipid-modifizierten Domäne an hydrophobe Oberflächen bindet und über eine zweite funktionalen Bindedomäne ein Ligandenmolekül auf der Oberfläche fixieren kann. Ein erheblicher Nachteil dieses Verfahrens besteht in der nichtkovalenten Bindung der Liganden. Weiterhin ist das Verfahren nur mit erheblichem Aufwand auf zahlreiche unterschiedliche Liganden übertragbar.

US 6,270,983 (Strohner et al.) beschreibt die Beschichtung von Oberflächen mit biotinylierten Molekülen indem die Oberfläche mit einem biotinylierten Trägerreagenz und einer aufgelagerten Avidin bzw. Streptavidinschicht vorbeschichtet wird. Aufgrund der Verwendung von Avidin bzw. Steptavidinschichten zur Bindung biotinylierter Liganden auf Oberflächen bleibt hier jedoch das Problem unspezifischer Interferenzen und eingeschränkter Beladungsdichte bestehen.

Cannale at al. (Biomacromolecules. 2011, 12, 3692-3697) entwickelten ein Verfahren zur Beschichtung von Mikrotiterplatten auf Basis einer 1,3-dipolaren Cycloadditionsreaktion zwischen Aziden und sterisch angespannten zyklischen Alkinen. Diese Verfahren erfolgt unter aggressiven chemischen Bedingungen und ist auf die Beschichtung von synthetischen Peptiden beschränkt.

Die bisherigen Verfahren zur Beschichtung von Oberflächen mit Molekülen weisen zahlreiche Einschränkungen in Bezug auf eine hohe Beladungsdichte, den Erhalt der biologischen Aktivität und einer einfachen Übertragbarkeit des Beschichtungsverfahrens auf verschiedene Moleküle auf. Diese Einschränkungen und Nachteile werden durch die vorliegende Erfindung behoben. Zudem besteht ein anhaltender Bedarf für Verfahren zur effizienten Beschichtung von Oberflächen mit biologisch aktiven Molekülen.

### Beschreibung

Die vorliegende Erfindung betrifft Verfahren zur adsorptiven Bindung von biologisch aktiven Molekülen auf Oberflächen indem die Oberfläche in einem ersten Schritt mit einem Trägerreagenz beschichtet wird und die zu bindenden biologisch aktiven Moleküle anschließend durch eine biorthogonale Cycloadditionsreaktion kovalent an das Trägerreagenz gekoppelt werden. Anwendungsgebiete der Erfindung sind die biochemische Forschung, die medizinische Diagnostik und die pharmazeutische Industrie.

Durch die vorliegende Erfindung werden die im Stand der Technik bestehenden Einschränkungen bei der Bindung von Molekülen auf Oberflächen in Bezug auf Proteindenaturierung, sterische Blockierung, unspezifische Interferenzreaktionen und geringe Bindungsdichte überwunden. Zusätzlich wird eine einfache einheitliche Methode zur stabilen und spezifischen Bindung unterschiedlicher Moleküle auf verschiedenen Oberflächen zur Verfügung gestellt. Die vorliegende Erfindung ermöglicht die Beschichtung von Oberflächen mit Molekülen in biologisch aktiver Form für die biochemische Forschung und für die Verwendung in diagnostischen Testsystemen.

In einem ersten Aspekt stellt die vorliegende Erfindung ein Verfahren zur Anbindung biologisch aktiver Moleküle an einer Oberfläche eines Substrats bereit, umfassend die Schritte
(i) Bereitstellen eines Trägermoleküls umfassend zumindest eine Click funktionelle Gruppe,
(ii) adsorptives Anbinden des Trägermoleküls an einer Oberfläche eines Substrats, und
(iii) Anbinden eines biologisch aktiven Moleküls umfassend zumindest eine gegenüber der Click-funktionellen Gruppe des Trägermoleküls reaktive Click-funktionelle Gruppe durch Cycloadditionsreaktion zwischen den miteinander reaktiven Click-funktionellen Gruppen.

Mit dem Verfahren der vorliegenden Erfindung gelingt es, an die Oberfläche eines grundsätzlich beliebigen festen Substrats biologisch aktive Moleküle anzubinden. Die Anbindung erfolgt dabei über ein Trägermolekül.

In Schritt (i) des erfindungsgemäßen Verfahrens wird ein Trägermolekül bereitgestellt, welches zumindest eine Click-funktionelle Gruppe umfasst. Unter einem "Trägermolekül" wird erfindungsgemäß ein Molekül verstanden, welches einerseits zur Anbindung an einer Oberfläche eines festen Substrats in der Lage ist und andererseits zumindest eine Click-funktionelle Gruppe aufweist. Vorzugsweise umfasst das Trägermolekül ein Makromolekül, ausgewählt aus Polypeptiden, Proteinen, Glycoproteinen und Kohlenhydraten. Das Molekulargewicht des Trägermoleküls liegt vorzugsweise bei ≥ 10 kDa. Besonders bevorzugt umfasst das Trägermolekül humanes Serumalbumin oder Rinderserumalbumin.

Eine "Click-funktionelle Gruppe" im Sinne der vorliegenden Erfindung bezeichnet eine funktionelle Gruppe, die in einer Cycloadditionsreaktion mit einer weiteren Click-funktionellen Gruppe zur Reaktion gebracht werden kann. Die Cycloadditionsreaktion zwischen Click-funktionellen Gruppen wird im Folgenden auch als Click-Reaktion bezeichnet.

Ein erfindungsgemäßes Trägermolekül kann in einer ersten Ausführungsform eine Click-funktionelle Gruppe umfassen, bei der es sich um eine Click-funktionelle ungesättigte Gruppe handelt. Beispiele hierfür sind Dipolarophile wie beispielsweise Alkene und Alkine, sowie Moleküle, die verwandte Heteroatom-funktionelle Gruppen umfassen, wie beispielsweise Carbonyle und Nitrile. Besonders bevorzugte Beispiele für Click-reaktive ungesättigte Gruppen sind Alkine.

In einer weiteren Ausführungsform kann ein Trägermolekül im Sinne der Erfindung eine Click-funktionelle dipolare Gruppe umfassen. Unter Click-funktionellen dipolaren Gruppen werden erfindungsgemäß solche Verbindungen verstanden, die ein oder mehrere Heteroatome umfassen und die zumindest eine mesomere Struktur aufweisen, die einen geladenen Dipol darstellt. Bevorzugt sind als Click-funktionelle dipolare Gruppen lineare 1,3-dipolare Gruppen, z. B. Azid, Nitriloxid, Diazoalkan, Nitrilimin und Nitron.

Andere Click-funktionelle Gruppen, die als Reaktionspartner für Click-funktionelle ungesättigte Gruppen dienen können, sind Diene wie z.B. Tetrazin und Tetrazol. Entsprechend kann ein Trägermolekül im Sinne der vorliegenden Erfindung eine Click-funktionelle Dien-Gruppe umfassen, insbesondere Tetrazin oder Tetrazol.

In Schritt (ii) des erfindungsgemäßen Verfahrens wird das Trägermolekül an einer Oberfläche eines Substrats gebunden. Die Anbindung erfolgt adsorptiv Abhängig von der zu beschichtenden Oberfläche können geeignete Trägermoleküle gewählt werden, die zur einer adsorptiven Bindung in der Lage sind.

Über die Click-funktionelle Gruppe des Trägermoleküls wird in Schritt (iii) ein biologisch aktives Molekül angebunden, wobei dieses biologisch aktive Molekül zumindest eine gegenüber der Click-funktionellen Gruppe des Trägermoleküls reaktive Click-funktionelle Gruppe aufweist. Die Umsetzung in Schritt (iii) umfasst beispielsweise eine Inkubation unter geeigneten Bedingungen.

Die Click-funktionellen Gruppen des Trägermoleküls und des biologisch aktiven Moleküls sind so gewählt, dass sie als Reaktionspartner in einer Cycloadditionsreaktion miteinander umgesetzt werden können. Durch die Cycloadditionsreaktion wird eine cyclische, beispielsweise heterocyclische Verknüpfung zwischen dem biologisch aktiven Molekül und dem bereits an der Oberfläche eines Substrats angebunden Trägermolekül geschlossen. Ein "biologisch aktives Molekül" im Sinne der Erfindung bezeichnet grundsätzlich beliebige biologisch aktive Moleküle. Beispiele für biologisch aktive Moleküle sind Proteine, Enzyme, Antikörper und Nukleinsäuren sowie Liganden dieser Moleküle.

Als "Liganden" werden beliebige Moleküle bezeichnet, die in der Lage sind, an ein Zielmolekül, beispielsweise an ein Protein, einen Rezeptor, ein Enzym, einen Antikörper oder eine Nukleinsäure zu binden. Die Bindung des Liganden ist üblicherweise reversibel und wird beispielsweise durch Ionenbindungen, Wasserstoffbrückenbindungen, Van-der-Waals-Kräfte und hydrophobe Effekte ermöglicht.

Mit dem erfindungsgemäßen Verfahren gelingt es insbesondere, niedermolekulare biologisch aktive Moleküle so anzubinden, dass sie in für Reaktionspartner zugänglicher Form vorliegen und ihre biologische Aktivität bewahren. In einer besonders bevorzugten Ausführungsform ist ein biologisch aktives Molekül im Sinne der Erfindung eine niedermolekulare Verbindung, insbesondere ein niedermolekularer Ligand eines der vorgenannten Moleküle. Unter "niedermolekular" wird im Sinne der Erfindung ein Molekül verstanden mit einem Molekulargewicht von ≤ 800g/mol.

Biologisch aktive Moleküle im Sinne der Erfindung können beispielsweise natürliche und nicht natürliche Aminosäuren deren Oligomere oder Polymere sein, wie z.B. Peptide, Rotaxane, Glycopeptide, Proteine, Enzyme, Antiköper etc. Andere Beispiele für biologisch aktive Moleküle sind Nukleinsäuren und Nukleinsäure-analoge Moleküle wie beispielsweise DNA, RNA, LNA, PNA, MeO-RNA, Phosphorthioat Nukleinsäuren etc.

Ein erfindungsgemäßes biologisch aktives Molekül kann in einer ersten Ausführungsform eine Click-funktionelle Gruppe umfassen, bei der es sich um eine dipolare Gruppe oder eine Dien-Gruppe handelt, wie vorstehend im Zusammenhang mit Click-funktionellen Gruppen an Trägermolekülen definiert. Solche biologisch aktiven Moleküle können in dem erfindungsgemäßen Verfahren insbesondere an solche Trägermoleküle angebunden werden, die eine Click-funktionelle, ungesättigte Gruppe umfassen.

In einer weiteren Ausführungsform kann ein biologisch aktives Molekül im Sinne der Erfindung eine Click-funktionelle Gruppe umfassen, bei der es sich um eine ungesättigte Gruppe handelt, wie vorstehend im Zusammenhang mit Click-funktionellen Gruppen an Trägermolekülen definiert. Solche biologisch aktiven Moleküle sind insbesondere zur Anbindung an Trägermoleküle geeignet, die selbst eine Click-funktionelle dipolare Gruppe oder eine Click-funktionelle Dien-Gruppe umfassen.

Die Click-funktionelle Gruppe kann an das Trägermolekül bzw. an das biologisch aktive Molekül direkt oder über einen Linker angebunden sein. Ein Linker im Sinne der Erfindung kann eine Kettenlänge von etwa 1 bis 20 oder mehr Atomen aufweisen. Er kann flexibel sein, z.B. ein Alkylen-basierter Linker, welcher wahlweise Heteroatome wie O, S und/oder N umfassen kann, oder er kann zumindest teilweise steif sein, z.B. ein Linker, welcher zumindest eine steife Gruppe umfasst, die ausgewählt sein kann aus Alkylengruppen, Alkingruppen, cyclischen Gruppen, insbesondere aromatischen oder heteroaromatischen Gruppen, aber auch cycloaliphatischen Gruppen und Kombinationen davon.

Die Form des Substrats kann erfindungsgemäß beliebig gewählt werden. Beispielsweise kann es in partikulärer Form, beispielsweise als Mikro- oder Nanopartikel, vorliegen. Alternativ kann das Substrat in Form einer Platte, insbesondere einer Mikrotiterplatte vorliegen. Das Material des Substrats ist grundsätzlich nicht eingeschränkt. Beispielsweise kann das Substrat Kunststoff, insbesondere Polysterol, Polypropylen, Poethylen, Polyamid, Polymethacrylat, Polycarbonat, Polyacrylat oder Copolymere des Polysterols, Glas, Kermaik, Latex, Metall, insbesondere Gold und/oder Kohlenstoff umfassen.

Die Cycloadditionsreaktion in Schritt (iii) des erfindungsgemäßen Verfahrens kann unter grundsätzlich beliebigen für die Kopplung Click-funktioneller Gruppen geeigneten Reaktionsbedingungen durchgeführt werden. Vorzugsweise wird die Cycloadditionsreaktion in Gegenwart eines Katalysators durchgeführt. Verfahren zur Durchführung von Cycloadditionsreaktionen zwischen Click-funktionellen Gruppen sowie geeignete Reaktionsbedingungen und Katalysatoren sind im Fachbereich bekannt.

Vorzugsweise wird durch die Cycloadditionsreaktion zwischen den Click-funktionellen Gruppen ein fünf- oder sechsgliedriger Carbocyclus oder Heterocyclus gebildet. Wenn beispielsweise als Click-funktionelle Gruppen eine Alkingruppe mit einer Azidgruppe zur Reaktion gebracht werden, so führt die Cycloadditionsreaktion zur Bildung einer 1,2,3-Triazolgruppe als fünfgliedriger Heterocyclus. Vorzugsweise umfasst die Cycloadditionsreaktion eine (3+2) 1,3-dipolare Cycloaddition unter Bildung eines fünfgliedrigen Heterocyclus.

In einem weiteren Aspekt betrifft die vorliegende Erfindung einen Artikel, umfassend
(i) ein Substrat mit einer Oberfläche und
(ii) ein Trägermolekül,
wobei das Trägermolekül adsorptiv an die Oberfläche des Substrats gebunden ist und zumindest eine Click-funktionelle Gruppe umfasst.

Substrat, Trägermolekül und Click-funktionelle Gruppe sind wie vorstehend definiert.

Eine solches, mit Click-funktionalisierten Trägermolekülen vorbeschichtetes Substrat kann beispielsweise verwendet werden, um nach Bedarf weitere Moleküle durch Click-Reaktion anzubinden.

Ein weiterer Gegenstand der vorliegenden Erfindung ist ein Artikel, umfassend
(i) ein Substrat mit einer Oberfläche,
(ii) ein Trägermolekül und
(iii)ein biologisch aktives Molekül,
wobei das Trägermolekül adsorptiv an die Oberfläche des Substrats gebunden ist und das biologisch aktive Molekül über einen Linker an das Trägermolekül gebunden ist, der eine durch Click-Reaktion gebildete Gruppe enthält.

Substrat, Trägermolekül und biologisch aktives Molekül sind wie vorstehend definiert.

In dem erfindungsgemäßen Artikel ist biologisch aktives Molekül über einen Linker an das Trägermolekül gebunden, der eine durch Click-Reaktion gebildete Gruppe enthält. Als eine durch Click-Reaktion gebildete Gruppe wird eine solche Gruppe verstanden, die durch Cycloaddition zwischen komplementären, Click-funktionellen Gruppen gebildet wurde bzw. erhältlich ist. Diese Gruppe ist eine cyclische, z.B heterocyclische Gruppe wie beispielsweise ein 1,2,3-Triazolring.

In den erfindungsgemäßen Artikeln kann das an der Substratoberfläche adsorptiv gebundene Trägermolekül wahlweise quervernetzt oder nicht quervernetzt vorliegen. Untereinander quervernetzte Trägermoleküle bilden zu benachbarten, weiteren Trägermolekülen kovalente Bindungen aus.

Ein weiterer Gegenstand der vorliegenden Erfindung ist ein Kit umfassend
(i) ein Substrat mit einer Oberfläche,
(ii) ein Trägermolekül, das adsorptiv an die Oberfläche des Substrats gebunden ist und zumindest eine Click-funktionelle Gruppe aufweist, und
(iii) ein Reagenz, welches die Modifikation eines biologisch aktiven Moleküls mit einer Click-funktionellen Gruppe ermöglicht, die gegenüber der Click-funktionellen Gruppe des Trägermoleküls (ii) reaktiv ist.

In einem erfindungsgemäßen Kit sind Substrat, Trägermolekül und biologisch aktives Molekül wie vorstehend definiert.

Das Reagenz (iii) für die Modifikation eines biologisch aktiven Moleküls mit einer Click-funktionellen Gruppe umfasst eine Click-funktionelle Gruppe, die wahlweise in geschützter Form vorliegen kann. Gemäß der vorliegenden Erfindung kann unter dem Begriff "Reagenz" hier auch eine Kombination von Reagenzien verstanden werden, sofern für die Modifizierung eines biologisch aktiven Moleküls eine Kombination mehrerer Reagenzien benötigt wird oder vorteilhaft ist. Verfahren und Reagenzien für die Modifizierung biologisch aktiver Moleküle mit einer Click-funktionellen Gruppe sind im Fachbereich bekannt.

Ein weiterer Aspekt der Erfindung betrifft die Verwendung eines Trägermoleküls, welches zumindest eine Click-funktionelle Gruppe und ein Makromolekül ausgewählt aus Polypeptiden, Proteinen, Glycoproteinen und Kohlenhydraten umfasst und ein Molekulargewicht von ≥ 10 kDa aufweist, zur Herstellung eines erfindungsgemäßen Artikels, wie vorstehend definiert. Als Makromolekül ist humanes Serumalbumin und Rinderserumalbumin besonders bevorzugt.

Ein weiterer Aspekt der Erfindung betrifft die Verwendung eines erfindungsgemäßen Artikels zur Bestimmung eines Analyten in einer Probe. Die Bestimmung kann einen qualitativen Nachweis umfassen, z.B. die Bestimmung des Vorliegens oder der Abwesenheit eines Analyten in einer zu analysierenden Probe. Die Erfindung ermöglicht aber auch einen quantitativen Nachweis eines Analyten, in einer zu analysierenden Probe. Für einen qualitativen und/oder quantitativen Nachweis können Reportergruppen bestimmt werden. Solche Reportergruppen sind ebenso wie Verfahren zur deren Nachweis im Fachbereich bekannt.

Der zu bestimmende Analyt kann beispielsweise ein in einer biologischen Probe vorhandener Analyt sein, wie z.B. ein Biomolekül, Arzneistoff oder toxische Verbindungen. Beispiele für Biomoleküle als Analyten sind Nukleinsäuren, Peptide, Polypeptide, Saccharide, Lipide, Steroide etc. Beispielsweise kann der Analyt aus Nukleinsäuren und Nukleosid-, Nukleotid- oder Nukleinsäure-bindenden Molekülen, wie z.B. Nukleosid, Nukleotid oder Nukleotid-bindenen Proteinen ausgewählt sein.

Weiter bevorzugt ist der Analyt eine Nukleinsäure, die anhand im Fachbereich bekannter Verfahren, wie beispielsweise Hybridisierungstechniken, nachgewiesen werden kann. Weiter kann unter Verwendung eines erfindungsgemäßen Artikels ein einzelner Analyt in einer Probe nachgewiesen werden oder es können parallel mehrere unterschiedliche Analyten nachgewiesen werden. In einer solchen Ausführungsform umfasst ein erfindungsgemäßer Artikel vorzugsweise mehrere unterschiedliche biologisch aktive Moleküle. Diese können über unterschiedliche Linker an die auf der Substratoberfläche immobilisierten Trägermoleküle gebunden sein. Solche unterschiedlichen Linker umfassen beispielsweise jeweils unterschiedliche cyclische Gruppen, die durch Click-Reaktion erhältlich sind.

Die Probe in der unter Verwendung eines erfindungsgemäßen Artikels ein Analyt nachgewiesen werden soll, kann eine biologische Probe, z.B. eine landwirtschaftliche Probe oder eine klinische Probe, wie z.B eine Gewebeprobe oder eine Körperflüssigkeitsprobe sein, z.B. Blut, Serum, Plasma etc. Weitere Arten von Proben sind u.a. Bodenproben, Lebensmittelproben, forensische Proben oder Stoffproben, die auf Markenschutz hin untersucht werden sollten.

Im Weiteren soll die vorliegende Erfindung detaillierter durch die folgenden Beispiele und Zeichnungen beschrieben werden.

### Beispiel 1: Herstellung von Dibenzylcyclooctine-PEG5 modifiziertem Rinderserumalbumin als Trägerreagenz

Das folgende Beispiel beschreibt die Herstellung von Dibenzylcyclooctine-PEG5 (DBCO) modifiziertem Rinderserumalbumin (BSA) als reaktives Trägerreagenz zur Beschichtung von Oberflächen. Dibenzylcyclooctin-PEG5-N-hydroxysuccinimid wurde nach bekannter Methode präpariert (Ning , X. H. et al. (2008), Angew. Chemie-Int., 47: 2253-2255). Rinderserumalbumin wird in 50 mM HEPES (2-(4-(2-Hydroxyethyl)-1-piperazinyl)-ethansulfonsäure), pH 8,0, 150 mM NaCl gelöst und bei 22 °C für 60 Minuten mit einem 30-fachen molaren Überschuss von Dibenzylcyclooctyn-PEG5-N-hydroxysuccinimid umgesetzt. Überschüssiges Reagenz und niedermolekulare Reaktionsprodukte wurden durch mehrmalige Dialyse gegen 50 mM HEPES, pH 8,0 , 150 mM NaCl entfernt. Das Reaktionsprodukt als DBCO-BSA bezeichnet, wurde bis zur Verwendung gefroren bei -20 C gelagert.

### Beispiel 2: Herstellung von adsorptiv DBCO-BSA-Trägerreagenz beschichteten Mikrotiterplatten

Das folgende Beispiel beschreibt die Herstellung von DBCO-BSA beschichteten Mikrotiterplatten (MTP) als präaktivierte Oberfläche zur nachfolgenden kovalenten Kopplung von biologisch aktiven Molekülen durch biorthogonale Cycloaddition.

Das gemäß 1.1 hergestellte Trägerreagenz DBCO-BSA wurde in einer Konzentration von 5 µg/ml in 150 mM NaCl, 100 mM TrisHCl pH 8,0 verdünnt und in 100 µl Aliquots in die Kavitäten einer Mikrotiterplatte (Costar, Maxisorb) pipettiert. Nach Inkubation für 16 Stunden bei 4 °C wurden die Kavitäten entleert und mit 100 µl/Kavität einer 1 X PBS , 0, 5 % BSA, 0,5 % Sucroselösung befüllt und 1 h bei Raumtemperatur blockiert. Anschließend wurden die Platten entleert und 2 h bei 37 °C getrocknet. Die getrockneten Platten mit einer DBCO-BSA Trägerreagenz-Beschichtung wurden bis zur weiteren Verwendung in Gegenwart eines Silicagel Trockenmittels bei 4 °C gelagert.

### Beispiel 3: Herstellung von DBCO-BSA-Trägerreagenz beschichteten Nanopartikeln

Das folgende Beispiel beschreibt die Herstellung von paramagnetischen Mikropartikeln (MMP) die adsorptiv mit dem Trägerreagenz DBCO-BSA vorbeschichtet sind und eine präaktivierte Oberfläche zur nachfolgenden kovalenten Kopplung von biologisch aktiven Molekülen durch biorthogonale Cycloaddition zur Verfügung stellen .

Paramagnetische Nanopartikel (PMC-130, Kisker GmbH) wurden in einer Konzentration von 5,6 mg/ml in 10 mM Na-Morpholinoethanlsulfonsäurepuffer (MES) pH 5,9 suspendiert, mit 12,5 mg/ml N-Hydroxysuccinimid und 25 mg/ml *N***-Ethyl-*N'*-(3-dimethylaminopropyl)carbodiimid Hydrochlorid** versetzt und 20 Minuten bei Raumtemperatur inkubiert. Anschließend wurde der Reaktionsansatz mit 100 mM HCl auf pH 3,0 eingestellt. Die Nanopartikel wurden mit Hilfe eines Magneten pelletiert, in 150 mM NaCl gewaschen und anschließend in bei einer Konzentration von 5,6 mg/ml in 150 mM NaCl resuspendiert. Zur Kopplung einer BSA-Beschichtung wurde 1 ml der aktivierten Nanopartikelsuspension mit 1,5 ml 1 % BSA in 25 mM HEPES-Puffer pH 7,98 versetzt und 20 Stunden bei 4 °C inkubiert. Die Reaktion wurde durch Zugabe von 15 µl 2 M Ethanolamin pH 8,0 gequencht. Nach Inkubation für 1 h bei Raumtemperatur wurden die BSA-beschichteten Nanopartikel in 25 mM HEPES, 150 mM NaCl, pH 8,0 gewaschen und in 20 ml 25 mM HEPES, 150 mM NaCl, 20 % DMSO, 0,05 mg/ml Dibenzylcyclooctin-PEG5-N-hydroxysuccinimid resuspendiert und 3 h bei Raumtemperatur inkubiert. Die DBCO-modifizierten BSA-Nanopartikel wurden in 20 mM Na-HEPES, 1 % BSA, pH 8,0 gewaschen, und bei einer Partikelkonzentration von 10 mg/ml in 20 mM Na-HEPES, 1 % BSA, 0,05 % Proclin, pH 8,0 bis zur Verwendung bei 4 °C gelagert.

### Beispiel 4: Herstellung von Azid-modifizierter Proteinase-3

Die Herstellung von 8-Azid-3,6-dioxaoctansäure N-hydroxysuccinimidester erfolgte nach bekannter Methode (George W. Anderson , Joan E. Zimmerman , Francis M. Callahan, J. Am. Chem. Soc., 1964, 86 (9), pp 1839-1842). Proteinase 3 wurde in einer Konzentration von 1 mg/ml in 50 mM HEPES, pH 8,0, 500 mM NaCl gelöst und mit 10 µl/ml einer 8-Azid-3,6-dioxaoctansäure N-hydroxysuccinimidester Stammlösung (10 mg/ml in DMSO) versetzt. Der Reaktionsansatz wurde 60 Minuten bei 37 °C inkubiert. Überschüssige Reagenzien und niedermolekulare Reaktionsprodukte wurden durch Dialyse gegen in 50 mM Na-HEPES, pH 8,0, 500 mM NaCl entfernt. Die Azid-modifizierte Proteinase-3 wurde bis zur weiteren Verwendung bei -20 °C gelagert.

### Beispiel 5: Herstellung von Azid-modifizierter Myeloperoxidase

Myeloperoxidase wurde in einer Konzentration von 1 mg/ml in 50 mM Na-HEPES, pH 8,0 , 500 mM NaCl gelöst und mit 10 µl/ml einer 8-Azid-3,6-dioxaoctansäure N-hydroxysuccinimidester Stammlösung (10 mg/ml in DMSO) versetzt. Der Reaktionsansatz wurde 60 Minuten bei 37 C inkubiert , anschließend mit 0,1 µl/ml 10 % H₂O₂ und 0,1 µl/ml 10 % NaN₃ versetzt und weitere 60 Minuten bei 37 °C inkubiert. Überschüssige Reagenzien und niedermolekulare Reaktionsprodukte wurden durch Dialyse gegen in 50 mM Na-HEPES, pH 8,0 , 500 mM NaCl entfernt. Die Azid-modifizierte Myeloperoxidase wurde bis zur weiteren Verwendung bei -20 °C gelagert.

### Beispiel 6: Herstellung von Proteinase-3 beschichteten Mikrotiterplatten und Bestimmung von PR3-Autoantikörpern

Das folgende Beispiel beschreibt die Herstellung von Proteinase-3 (PR3) beschichteten Mikrotiterplatten als Antigenoberfläche zum Nachweis von PR3-Autoantikörpern in humanem Serum.

Die gemäß Beispiel 4 hergestellte Azid-modifizierte Proteinase-3 wurde in einer Konzentration von 0,5 µg/ml in Blockierungspuffer (1 X PBS, 0,5 % BSA, 1 % Laktose, 5 mM EDTA) verdünnt und in 100 µl Aliquots in die Kavitäten einer gemäß Beispiel 2 hergestellten DBCO-BSA-Trägerreagenz beschichteten Mikrotiterplatte pipettiert. Nach Inkubation für 16 Stunden bei 22 °C wurden die Kavitäten entleert, mit 100 µl/Kavität der Blockierungslösung befüllt und 1 h bei Raumtemperatur inkubiert. Hierbei erfolgte durch biorthogonale Cycloaddition eine spezifische kovalente Kopplung der Azid-modifizierten Proteinase-3 an die DBCO-BSA-Trägerreagenz vorbeschichtete Mikrotiterplatte. Anschließend wurden die Platten entleert und 2 h bei 37 °C getrocknet. Die getrockneten Platten mit einer PR3-Beschichtung wurden bis zur weiteren Verwendung in Gegenwart eines Silicagel Trockenmittels bei 4 °C gelagert.

Um die Eignung der PR3-Beschichtung zum Nachweis von PR3-Autoantikörpern in humanem Serum zu untersuchen wurde ein humanes Anti-PR3 Serum in einer Verdünnungsserie mit den Verdünnungsstufen 1:50, 1:100, 1:200, 1:400, 1:1000, 1:2000 in Probenpuffer eines PR3-Assays (ORG 518, ORGENTEC GmbH) verdünnt. Von serumfreien Probenpuffer und den Serumverdünnungen wurden je 100 µl in DBCO-BSA-PR3 beschichtete Mikrotiterkavitäten bzw. in antigenfreie DBCO-BSA-Kavitäten pipettiert und 30 min bei 20 - 25 °C inkubiert. Die Mikrotiterplatten wurden gewaschen und die Bindung von humanen PR3-Autoantikörpern wurde mit einem Anti-human-lgG-Peroxidase markierten Sekundärantikörper und einer Tetramethylbenzidin (TMB)-Farbreaktion durch Messung der optischen Dicht bei 450 nm bestimmt.

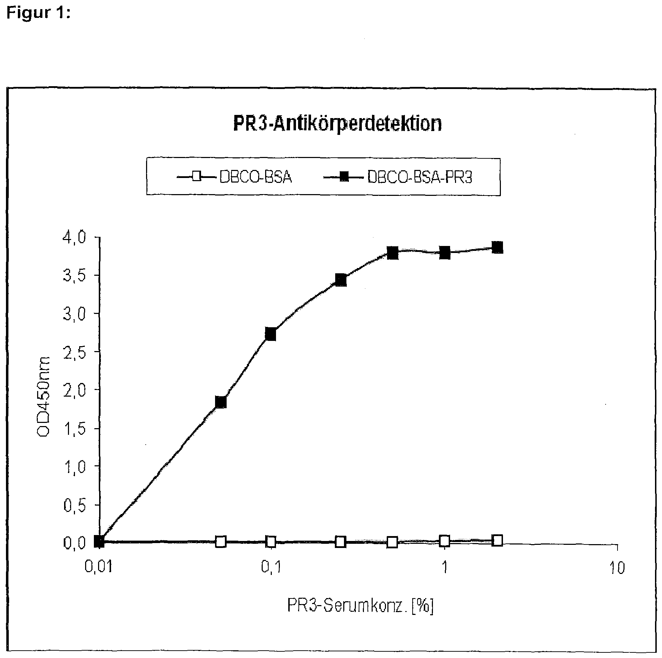

Hierbei konnten wie in Figur 1 dargestellt selbst bei einer 0,05 % Konzentration (1:2000 Verdünnung) des Testserums PR3-Autoantikörper spezifisch detektiert werden. Der Vergleich der Reaktionsniveaus der Serum-Verdünnungsstufen bei der Bindung an PR3-DBCO-BSA bzw. DBCO-BSA beschichtete Mikrotiterplatten zeigt, dass im Konzentrationsbereich von 0 - 2 % Serum PR3-Autoantikörper mit hoher Sensitivität und Spezifität detektiert wurden, während an der antigenfreien DBCO-BSA-Trägerbeschichtung nur ein geringes Hintergrundsignal auftrat.

### Beispiel 7: Herstellung von Myeloperoxidase beschichteten Mikrotiteroberflächen und Bestimmung von MPO-Autoantikörpern

Das folgende Beispiel beschreibt die Herstellung von Myeloperoxidase (MPO) beschichteten Mikrotiterplatten als Antigenoberfläche zum Nachweis von MPO-Autoantikörpern.

Die gemäß Beispiel 5 hergestellte Azid-modifizierte Myeloperoxidase wurde in einer Konzentration von 0,5 µg/ml in Blockierungspuffer (1 X PBS, 0,5 % BSA, 1 % Laktose, 5 mM EDTA) verdünnt und in 100 µl Aliquots in die Kavitäten einer gemäß Beispiel 2 hergestellten DBCO-BSA-Trägerreagenz beschichteten Mikrotiterplatte pipettiert. Nach Inkubation für 16 Stunden bei 22 °C wurden die Kavitäten entleert, mit 100 µl/Kavität des Blockierungspuffers befüllt und 1 h bei Raumtemperatur inkubiert. Anschließend wurden die Platten entleert und 2 h bei 37 °C getrocknet. Die getrockneten Platten mit einer MPO-Beschichtung wurden bis zur weiteren Verwendung in Gegenwart eines Silicagel Trockenmittels bei 4 °C gelagert.

Um die Eignung der MPO-Beschichtung zum Nachweis von MPO-Autoantikörpern in humanem Serum zu untersuchen wurde ein humanes Anti-MPO-Serum in einer Verdünnungsserie mit den Verdünnungsstufen 1:50, 1:100, 1:200, 1:400, 1:1000, 1:2000 in Probenpuffer eines MPO-Assays (ORG 519, ORGENTEC GmbH) verdünnt. Von serumfreien Probenpuffer und den Serumverdünnungen wurden je 100 µl in DBCO-BSA-MPO beschichtete Mikrotiterkavitäten bzw. in antigenfreie DBCO-BSA-Kavitäten pipettiert und 30 min bei 20 - 25 °C inkubiert. Die Mikrotiterplatten wurden gewaschen und die Bindung von humanen MPO-Autoantikörpern wurde mit einem Anti-human-lgG-Peroxidase markierten Sekundärantikörper und einer Tetramethylbenzidin (TMB)-Farbreaktion durch Messung der der optischen Dichte bei 450 nm bestimmt.

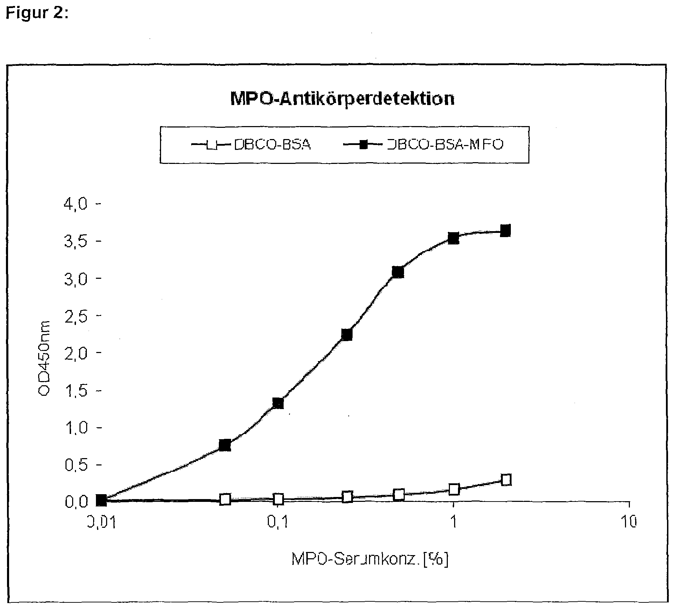

Hierbei konnten wie in Figur 2 dargestellt selbst bei einer 0,05 % Konzentration (1:2000 Verdünnung) des Testserums MPO-Autoantikörper spezifisch detektiert werden. Der Vergleich der Reaktionsniveaus der Serum-Verdünnungsstufen bei der Bindung an MPO-DBCO-BSA bzw. DBCO-BSA beschichtete Mikrotiterplatten zeigt, dass im Konzentrationsbereich von 0 - 2 % Serum, MPO-Autoantikörper mit hoher Sensitivität und Spezifität detektiert wurden, während an der antigenfreien DBCO-BSA-Trägerbeschichtung nur ein geringes Hintergrundsignal auftrat.

### Beispiel 8: Herstellung von Proteinase-3 beschichteten paramagnetischen Nanopartikeln und Bestimmung von PR3-Autoantikörpern

Das folgende Beispiel beschreibt die Herstellung von Proteinase-3 (PR3) beschichteten paramagnetischen Nanopartikeln und die Bestimmung von PR3-Autoantikörpern.

Azid-modifizierte Proteinase-3 gemäß Beispiel 4 wurde in einer Konzentration von 40 µg/ml in Blockierungspuffer (20 mM Na-HEPES, pH 8,0 , 1 % BSA) verdünnt und in Gegenwart von 2 % gemäß Beispiel 3 hergestellten DBCO-BSA-Trägerreagenz beschichteten Nanopartikeln für 16 Stunden bei 22 °C inkubiert. Die paramagnetischen Nanopartikel wurden in 1, 5ml Blockierungspuffer gewaschen und bei einer Partikelkonzentration von 2 % in 20 mM Na-HEPES, pH 8,0 , 1 % BSA, 0,05 % Proclin bei 4 °C bis zur Verwendung gelagert.

Um die Eignung der PR3-beschichteten Nanopartikel zum Nachweis von PR3-Autoantikörpern in humanem Serum zu untersuchen wurde ein humanes Anti-PR3 Serum in einer Verdünnungsserie mit den Verdünnungsstufen 1:50, 1:100, 1:200, 1:400, 1:800, 1:1600, 1:3200 in Probenpuffer eines PR3-Assays (ORG 518, ORGENTEC GmbH) verdünnt. Von serumfreiem Probenpuffer und Serumverdünnungen wurden je 100 µl mit 5 µl der PR3 beschichteten Nanopartikel versetzt und 30 min bei 20 - 25 °C inkubiert. Die Partikel wurden im Magnetfeld pelletiert und gewaschen. Die Bindung von humanen PR3-Autoantikörpern wurde mit einem Anti-human-lgG-Peroxidase markierten Sekundärantikörper und einer Tetramethylbenzidin (TMB)-Farbreaktion durch Messung der optischen Dichte bei 450 nm bestimmt.

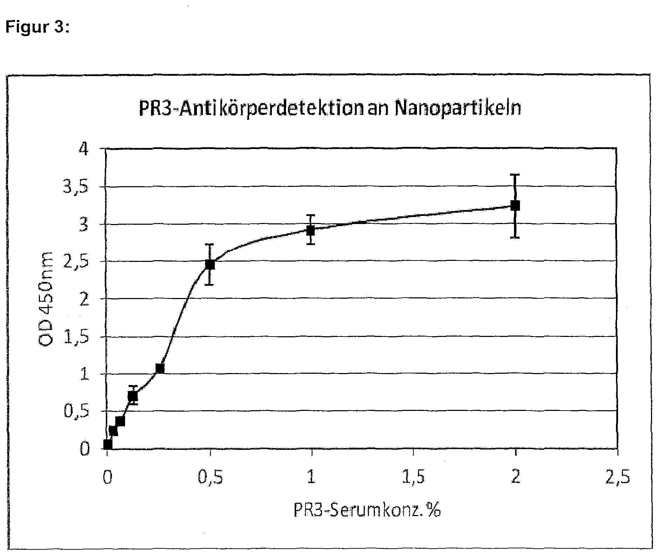

Hierbei konnten wie in Figur 3 dargestellt selbst bei einer 0,03 % Konzentration (1: 3200 Verdünnung) des Testserums PR3-Autoantikörper spezifisch detektiert werden.

### Beispiel 9: Herstellung von 8-Azid-3,6-dioxaoctansäure modifiziertem Rinderserumalbumin als Trägerreagenz

Das folgende Beispiel beschreibt die Herstellung von 8-Azid-3,6-dioxaoctansäure modifiziertem Rinderserumalbumin als reaktives Trägerreagenz zur Beschichtung von Oberflächen. Rinderserumalbumin wurde in einer Konzentration von 20 mg/ml in 20 mM HEPES, pH 7,5,120 mM NaCl gelöst und bei 22 °C für 60 Minuten mit einem 30-fachen molaren Überschuss von 8-Azid-3,6-dioxaoctansäure N-hydroxysuccinimidester (IRIS Biotech) umgesetzt. Überschüssiges Reagenz und kleinmolekulare Reaktionsprodukte wurden durch mehrmalige Dialyse gegen 20 mM HEPES, pH 7,5, 120 mM NaCl entfernt. Das Reaktionsprodukt als Azid-BSA bezeichnet, wurde bis zur Verwendung gefroren bei -20 C gelagert.

### Beispiel 10: Herstellung von Azid-BSA-Trägerreagenz beschichteten Nanopartikeln

Das folgende Beispiel beschreibt die Herstellung von paramagnetischen Mikropartikeln (MMP) die mit dem Trägerreagenz Azid-BSA vorbeschichtet sind.

Paramagnetische Nanopartikel (EM1 100/40, ESTAPOR) wurden in einer Konzentration von 3,3 mg/ml in 10 mM Na-Morpholinoethansulfonsäurepuffer (MES) pH 5,7 suspendiert, mit 0,70 mg/ml Azid-BSA und 0.58 mg/ml N-Ethyl-N'-(3-dimethylaminopropyl)carbodiimid Hydrochlorid versetzt und 180 Minuten bei Raumtemperatur inkubiert. Die Nanopartikel wurden mit Hilfe eines Magneten pelletiert, und in 25 mM HEPES, 150 mM NaCl, pH 8,0 gewaschen. Die Azido-modifizierten BSA-Nanopartikel wurden in 20 mM Na-HEPES, 1 % BSA, pH 8,0 gewaschen, und bei einer Partikelkonzentration von 10 mg/ml in 20 mM Na-HEPES, 1 % BSA, 0,05 % Proclin, pH 8,0 bis zur Verwendung bei 4 °C gelagert.

### Beispiel 11: Herstellung von DBCO-modifiziertem ß2-Glycoprotein

Humanes ß2-Glycoprotein wurde in einer Konzentration von 2.5 mg/ml in 20 mM Na-MES, pH 5,5, 120 mM NaCl gelöst und mit 9 µl/ml einer Dibenzylcyclooctin-PEG5-N-hydroxysuccinimid (10 mg/ml in DMSO) versetzt. Der Reaktionsansatz wurde 16 Stunden bei 4° C inkubiert. Überschüssige Reagenzien und kleinmolekulare Reaktionsprodukte wurden durch Dialyse gegen in 20 mM Na-HEPES, pH 8,0 ,120 mM NaCl entfernt. Das DBCO-modifizierte ß2-Glycoprotein wurde bis zur weiteren Verwendung bei -20 °C gelagert.

### Beispiel 12: Herstellung von DBCO-modifiziertem Cardiolipin

Eine 10 ml einer 0,5 % Cardiolipinlösung in Dichlormethan wurde mit 5 mg meta-Chlorperbenzoesäure versetzt und 24 h bei 4° C inkubiert. Nach Zusatz von 10 ml Dichlormethan wurde die Mischung 2 X mit je 10 ml 10% Natriumcarbonat extrahiert. Die Dichlormethanfraktion wurden über MgSO4 getrocknet, mit 0,2 ml 1,2-Ethylendiamine versetzt und 16 h bei Raumtemperatur inkubiert. Der Reaktionansatz wurde 3 X mit je 10 ml 10% Zitronensäure extrahiert. Die organische Phase enthält Amino-Cardiolipin und wurde unter Vakuum zu einem farblosen Öl konzentriert. Amino-Cardiolipin wurden in 10.0 ml Dichlormethan gelöst, mit 2 µl Triethylamine und 6 mg Dibenzylcyclooctin-PEG5-N-hydroxysuccinimid versetzt und 16 h bei Raumtemperatur umgesetzt. Das Lösungsmittel wurde unter Unterdruck abgedampft und der Rückstand wurde über Silicagelsäulechromatographie gereinigt. Hierbei wurden 50.0 mg von DBCO-(PEG)5-Cardiolipin in Form eines farblosen Wachses erhalten.

### Beispiel 13: Herstellung von Cardiolipin/ß2-Glycoprotein beschichteten paramagnetischen Nanopartikeln und Bestimmung von Cardiolipin/ß2-Glycoprotein-Autoantikörpern

Azid-BSA-Trägerreagenz beschichteten Nanopartikeln gemäß Beispiel 10 wurden als 2 % Suspension in Blockierungspuffer (20 mM Na-HEPES, pH 8,0, 120 mM NaCl, 1 % BSA) verdünnt und in Reaktionsansätzen mit DBCO-ß2-Glycoprotein (80 µg/ml) bzw. einer Mischung aus DBCO-ß2-Glycoprotein (80 µg/ml) und DBCO-Cardiolipin (40 µg/ml) versetzt und für 2 Stunden bei 22 °C inkubiert. Die mit ß2-Glycoprotein bzw. mit einer Mischung aus ß2-Glycoprotein und Cardiolipin kovalent beladenen paramagnetischen Nanopartikel wurden in Blockierungspuffer gewaschen und bei einer Partikelkonzentration von 2 % in 20 mM Na-HEPES, pH 8,0 , 1 % BSA, 0,05 % Proclin bei 4 °C gelagert.

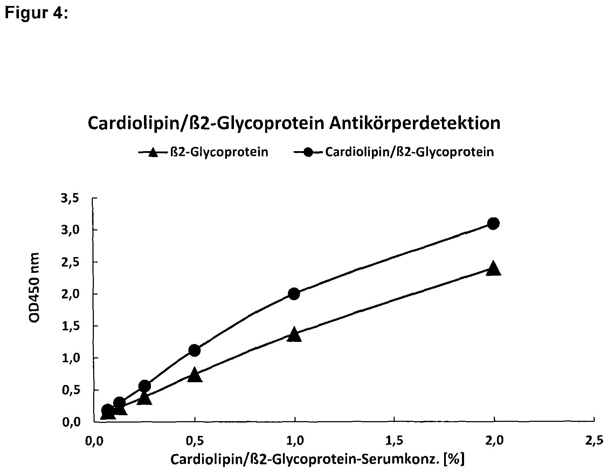

Um die Eignung der Cardiolipin/ß2-Glycoprotein Nanopartikel zum Nachweis von Cardiolipin/ß2-Glycoprotein-Autoantikörpern in humanem Serum zu untersuchen wurde ein humanes Anti-Cardiolipin/ß2-Glycoprotein Serum in einer Verdünnungsserie mit den Verdünnungsstufen 1:100, 1:200, 1:400, 1:800, 1:1600, 1:3200 in Probenpuffer eines Cardiolipin/ß2-Glicoprotein-Assays (ORG 515, ORGENTEC GmbH) verdünnt. Von serumfreiem Probenpuffer und Serumverdünnungen wurden je 100 µl mit 5 µl der Cardiolipin/ß2-Glycoprotein beschichteten Nanopartikel versetzt und 30 min bei 20 - 25 °C inkubiert. Die Partikel wurden im Magnetfeld pelletiert und gewaschen. Die Bindung von humanen Cardiolipin/ß2-Glycoprotein-Autoantikörpern wurde mit einem Anti-human-lgG-Peroxidase markierten Sekundärantikörper und einer Tetramethylbenzidin (TMB)-Farbreaktion durch Messung der optischen Dichte bei 450 nm bestimmt. Hierbei konnten wie in Figur 4 dargestellt Cardiolipin/ß2-Glycoprotein Autoantikörper spezifisch detektiert werden. Bei den Nanopartikeln die mit beiden Antigenen, Cardiolipin und ß2-Glycoprotein, belegt sind, ist ein additiver Effekt der Reaktivität sichtbar.

### Beschreibung der Abbildungen

**Figur 1****:**
   Bestimmung von PR3-Autoantikörpern in humanem Serum an DBCO-BSA-PR3 beschichteten Mikrotiterplatten . Verdünnungen eines humanen Serums mit PR3-Autoantikörpern in den angezeigten Konzentrationen wurden in DBCO-BSA-PR3 beschichteten Mikrotiterkavitäten und im Vergleich in antigenfreien DBCO-BSA beschichteten Mikrotiterkavitäten untersucht. Hierbei wurde die Bindung von PR3-Autoantikörpern an die Kavitätenoberfläche mit einem Peroxidase-markierten Anti-human-IgG Antikörper und einer TMB-Farbreaktion durch Messung der optischen Dichte bei 450 nm bestimmt.
**Figur 2****:**
   Bestimmung von MPO-Autoantikörpern in humanem Serum an DBCO-BSA-MPO beschichteten Mikrotiterplatten. Verdünnungen eines humanen Serums mit MPO-Autoantikörpern in den angezeigten Konzentrationen wurden in DBCO-BSA-MPO beschichteten Mikrotiterkavitäten und im Vergleich in antigenfreien DBCO-BSA beschichteten Mikrotiterkavitäten untersucht. Hierbei wurde die Bindung von MPO-Autoantikörpern an die Kavitätenoberfläche mit einem Peroxidase-markierten Anti-human-IgG Antikörper und einer TMB-Farbreaktion durch Messung der optischen Dichte bei 450 nm bestimmt.
**Figur 3****:**
   Bestimmung von PR3-Autoantikörpern in humanem Serum DBCO-BSA-PR3 beschichteten paramagnetischen Nanopartikeln. Verdünnungen eines humanen Serums mit PR3-Autoantikörpern in den angezeigten Konzentrationen wurden -BSA-PR3 beschichteten paramagnetischen Nanopartikeln versetzt Hierbei wurde die Bindung von PR3-Autoantikörpern an die Nanopartikel mit einem Peroxidase-markierten Anti-human-IgG Antikörper und einer TMB-Farbreaktion durch Messung der optischen Dichte bei 450 nm bestimmt.
**Figur 4****:**
   Bestimmung von Cardiolipin/ß2-Glycoprotein-Autoantikörpern in humanem Serum mit Azid-BSA-ß2-Glycoprotein bzw. mit Azid-BSA-Cardiolipin-ß2-Glycoprotein beschichteten paramagnetischen Nanopartikeln. Verdünnungen eines humanen Serums mit Cardiolipin/ß2-Glycoprotein-Autoantikörpern in den angezeigten Konzentrationen wurden mit beschichteten paramagnetischen Nanopartikeln versetzt Hierbei wurde die Bindung von Cardiolipin/ß2-Glycoprotein-Autoantikörpern an die Nanopartikel mit einem Peroxidase-markierten Anti-human-IgG Antikörper und einer TMB-Farbreaktion durch Messung der optischen Dichte bei 450 nm bestimmt.

## Patentansprüche

1. Verfahren zur Anbindung biologisch aktiver Moleküle an einer Oberfläche eines Substrats, umfassend die Schritte
(i) Bereitstellen eines Trägermoleküls umfassend zumindest eine Click-funktionelle Gruppe, wobei das Trägermolekül ein Makromolekül ausgewählt aus Polypeptiden, Proteinen, Glycoproteinen und Kohlenhydraten umfasst und ein Molekulargewicht von ≥10 kDA aufweist,
(ii) Anbinden des Trägermoleküls an einer Oberfläche eines Substrats, wobei das Trägermolekül adsorptiv an die Oberfläche des Substrats gebunden wird,
(iii) Anbinden eines biologisch aktiven Moleküls umfassend zumindest eine gegenüber der Click-funktionellen Gruppe des Trägermoleküls reaktive Click-funktionelle Gruppe an das Trägermolekül durch Cycloadditionsreaktion zwischen den miteinander reaktiven Click-funktionellen Gruppen.

2. Verfahren nach Anspruch 1, wobei die Click-funktionellen Gruppen ausgewählt sind aus Azid-, Alken-, Alkin-, Nitron-, Nitrilimin- Nitriloxid-, Isonitril-, Tetrazol- und Tetrazin-Gruppen.

3. Verfahren nach einem der vorhergehenden Ansprüche, wobei das Substrat Kunststoff, insbesondere Polystyrol, Polypropylen, Polyethylen, Polyamid, Polymethacrylat, Polycarbonat, Polyacrylat, oder Copolymere des Polystyrols, Glas, Keramik, Latex, Metall, insbesondere Gold, und/oder Kohlenstoff umfasst.

4. Verfahren nach einem der vorhergehenden Ansprüche, wobei das Substrat in Form einer Platte, insbesondere einer Microtiterplatte oder als Micro- oder Nanopartikel vorliegt.

5. Verfahren nach einem der vorhergehenden Ansprüche, wobei das Trägermolekül humanes Serumalbumin oder Rinderserumalbumin umfasst.

6. Verfahren nach einem der vorhergehenden Ansprüche, wobei das biologisch aktive Molekül ausgewählt ist aus Proteinen, Enzymen, Antikörpern, Nukleinsäuren und niedermolekularen Liganden von Proteinen, Enzymen, Antikörpern oder Nukleinsäuren.

7. Artikel, umfassend
(i) ein Substrat mit einer Oberfläche und
(ii) ein Trägermolekül,
wobei das Trägermolekül ein Makromolekül ausgewählt aus Polypeptiden, Proteinen, Glycoproteinen und Kohlenhydraten umfasst und ein Molekulargewicht von ≥10 kDA aufweist und wobei das Trägermolekül adsorptiv an die Oberfläche des Substrats gebunden ist und zumindest eine Click-funktionelle Gruppe umfasst.

8. Artikel, umfassend
(i) ein Substrat mit einer Oberfläche,
(ii) ein Trägermolekül und
(iii) ein biologisch aktives Molekül,
wobei das Trägermolekül ein Makromolekül ausgewählt aus Polypeptiden, Proteinen, Glycoproteinen und Kohlenhydraten umfasst und ein Molekulargewicht von ≥10 kDA aufweist und wobei das Trägermolekül adsorptiv an die Oberfläche des Substrats gebunden ist und das biologisch aktive Molekül über einen Linker an das Trägermolekül gebunden ist, der eine durch Click-Reaktion gebildete Gruppe enthält.

9. Artikel nach Anspruch 8, wobei die durch Click-Reaktion gebildete Gruppe ausgewählt ist aus 5-gliedrigen oder 6-gliedrigen Heterocyclen, insbesondere 1,2,3-Triazol.

10. Artikel nach einem der Ansprüche 7-9, wobei das Trägermolekül quervernetzt oder nicht quervernetzt ist.

11. Kit, umfassend
(i) ein Substrat mit einer Oberfläche,
(ii) ein Trägermolekül, das adsorptiv an die Oberfläche des Substrats gebunden ist und zumindest eine Click-funktionelle Gruppe aufweist, wobei das Trägermolekül ein Makromolekül ausgewählt aus Polypeptiden, Proteinen, Glycoproteinen und Kohlenhydraten umfasst und ein Molekulargewicht von ≥10 kDA aufweist, und
(iii) ein Reagenz, welches die Modifikation eines biologisch aktiven Moleküls mit einer Click-funktionellen Gruppe ermöglicht, die gegenüber der Click-funktionellen Gruppe des Trägermoleküls (ii) reaktiv ist.

12. Verwendung eines Trägermoleküls welches zumindest eine Click-funktionelle Gruppe und ein Makromolekül ausgewählt aus Polypeptiden, Proteinen, Glycoproteinen und Kohlenhydraten, insbesondere humanes Serumalbumin oder Rinderserumalbumin umfasst und ein Molekulargewicht von ≥ 10kDA aufweist, zur Herstellung eines Artikels nach einem der Ansprüche 7-10 oder eines Kits nach Anspruch 11.

13. Verwendung eines Artikels nach einem der Ansprüche 7-10, zur Bestimmung eines Analyten in einer Probe.

## Claims

1. Method for binding biologically active molecules to a surface of a substrate, comprising the steps of:
(i) providing a carrier molecule comprising at least one click functional group, wherein the carrier molecule comprises a macromolecule selected from polypeptides, proteins, glycoproteins and carbohydrates and has a molecular weight of ≥ 10 kDA,
(ii) binding the carrier molecule to a surface of a substrate, wherein the carrier molecule is adsorptively bound to the surface of the substrate,
(iii) binding a biologically active molecule comprising at least one click functional group that is reactive towards the click functional group of the carrier molecule to the carrier molecule by cycloaddition reaction between the mutually reactive click functional groups.

2. Method according to claim 1, wherein the click functional groups are selected from azide, alkene, alkyne, nitrone, nitrilimine, nitrile oxide, isonitrile, tetrazole and tetrazine groups.

3. Method according to any of the preceding claims, wherein the substrate comprises plastics material, in particular polystyrene, polypropylene, polyethylene, polyamide, polymethacrylate, polycarbonate, polyacrylate or copolymers of polystyrene, glass, ceramics, latex, metal, in particular gold, and/or carbon.

4. Method according to any of the preceding claims, wherein the substrate is in the form of a plate, in particular a microplate, or in the form of microparticles or nanoparticles.

5. Method according to any of the preceding claims, wherein the carrier molecule comprises human serum albumin or bovine serum albumin.

6. Method according to any of the preceding claims, wherein the biologically active molecule is selected from proteins, enzymes, antibodies, nucleic acids and low molecular weight ligands of proteins, enzymes, antibodies or nucleic acids.

7. Article, comprising
(i) a substrate having a surface, and
(ii) a carrier molecule,
wherein the carrier molecule comprises a macromolecule selected from polypeptides, proteins, glycoproteins and carbohydrates and has a molecular weight of ≥ 10 kDA and wherein the carrier molecule is adsorptively bound to the surface of the substrate and comprises at least one click functional group.

8. Article, comprising
(i) a substrate having a surface,
(ii) a carrier molecule, and
(iii) a biologically active molecule,
wherein the carrier molecule comprises a macromolecule selected from polypeptides, proteins, glycoproteins and carbohydrates and has a molecular weight of ≥ 10 kDA and wherein the carrier molecule is adsorptively bound to the surface of the substrate, and the biologically active molecule is bound to the carrier molecule via a linker which contains a group formed by click reaction.

9. Article according to claim 8, wherein the group formed by click reaction is selected from 5-membered or 6-membered heterocycles, in particular 1,2,3-triazole.

10. Article according to any of claims 7 to 9, wherein the carrier molecule is cross-linked or is not cross-linked.

11. Kit, comprising
(i) a substrate having a surface,
(ii) a carrier molecule which is adsorptively bound to the surface of the substrate and has at least one click functional group, wherein the carrier molecule comprises a macromolecule selected from polypeptides, proteins, glycoproteins and carbohydrates and has a molecular weight of ≥ 10 kDA and
(iii) a reagent which allows a biologically active molecule to be modified with a click functional group which is reactive towards the click functional group of the carrier molecule (ii).

12. Use of a carrier molecule which comprises at least one click functional group and a macromolecule selected from polypeptides, proteins, glycoproteins and carbohydrates, in particular human serum albumin or bovine serum albumin, and has a molecular weight of ≥ 10 kDa, for the production of an article according to any of claims 7 to 10 or of a kit according to claim 11.

13. Use of an article according to any of claims 7 to 10 for determining an analyte in a sample.

## Revendications

1. Procédé de liaison de molécules biologiquement actives à une surface d'un substrat, comprenant les étapes de
(i) mise à disposition d'une molécule porteuse comprenant au moins un groupe à fonction click, dans lequel la molécule porteuse comprend une macromolécule choisie parmi des polypeptides, des protéines, des glycoprotéines et des hydrates de carbone et présente un poids moléculaire ≥ 10 kDA,
(ii) liaison de la molécule porteuse à une surface d'un substrat, dans lequel la molécule porteuse est liée par adsorption à la surface du substrat,
(iii) liaison d'une molécule biologiquement active comprenant au moins un groupe à fonction click réactif par rapport au groupe à fonction click de la molécule porteuse à la molécule porteuse par réaction par cycloaddition entre les groupes à fonction click réactif les uns avec les autres.

2. Procédé selon la revendication 1, dans lequel les groupes à fonction click sont choisis parmi des groupes d'azide, d'alcène, d'alkine, de nitrone, d'imine de nitrile, d'oxyde de nitrile, d'isonitrile, de tétrazole et de tétrazine.

3. Procédé selon l'une quelconque des revendications précédentes, dans lequel le substrat comprend une matière plastique, en particulier du polystyrène, du polypropylène, du polyéthylène, du polyamide, du polyméthacrylate, du polycarbonate, du polyacrylate ou des copolymères du polystyrène, du verre, de la céramique, du latex, du métal, en particulier de l'or, et/ou du carbone.

4. Procédé selon l'une quelconque des revendications précédentes, dans lequel le substrat est présent sous la forme d'une plaque, en particulier d'une plaque microtitre ou en tant que micro- ou nanoparticules.

5. Procédé selon l'une quelconque des revendications précédentes, dans lequel la molécule porteuse comprend de l'albumine sérique humaine ou de l'albumine sérique bovine.

6. Procédé selon l'une quelconque des revendications précédentes, dans lequel la molécule biologiquement active est choisie parmi des protéines, des enzymes, des anticorps, des acides nucléiques et des ligands à faible poids moléculaire de protéines, d'enzymes, d'anticorps ou d'acides nucléiques.

7. Article comprenant
(i) un substrat avec une surface et
(ii) une molécule porteuse,
dans lequel la molécule porteuse comprend une macromolécule choisie parmi des polypeptides, des protéines, des glycoprotéines et des hydrates de carbone et présente un poids moléculaire ≥ 10 kDA et dans lequel la molécule porteuse est liée par adsorption à la surface du substrat et comprend au moins un groupe à fonction click.

8. Article comprenant
(i) un substrat avec une surface,
(ii) une molécule porteuse et
(iii) une molécule biologiquement active,
dans lequel la molécule porteuse comprend une macromolécule choisie parmi des polypeptides, des protéines, des glycoprotéines et des hydrates de carbone et présente un poids moléculaire ≥10 kDA et dans lequel la molécule porteuse est liée par adsorption à la surface du substrat et la molécule biologiquement active est liée à la molécule porteuse par un chaînon de liaison, qui contient un groupe formé par une réaction click.

9. Article selon la revendication 8, dans lequel le groupe formé par réaction click est choisi parmi des hétérocycles à 5 chaînons ou à 6 chaînons, en particulier un 1,2,3-triazole.

10. Article selon l'une quelconque des revendications 7 - 9, dans lequel la molécule porteuse est réticulée ou n'est pas réticulée.

11. Ensemble comprenant
(i) un substrat avec une surface,
(ii) une molécule porteuse, qui est liée par adsorption à la surface du substrat et présente au moins un groupe à fonction click, dans lequel la molécule porteuse comprend une macromolécule choisie parmi des polypeptides, des protéines, des glycoprotéines et des hydrates de carbone et présente un poids moléculaire ≥ 10 kDA, et
(iii) un réactif, qui permet la modification d'une molécule biologiquement active avec un groupe à fonction click, qui est réactif par rapport au groupe à fonction click de la molécule porteuse (ii).

12. Utilisation d'une molécule porteuse, qui comprend au moins un groupe à fonction click et une macromolécule choisie parmi des polypeptides, des protéines, des glycoprotéines et des hydrates de carbone, en particulier de l'albumine sérique humaine ou de l'albumine sérique bovine et présente un poids moléculaire ≥ 10 kDA, pour fabriquer un article selon l'une quelconque des revendications 7 - 10 ou un ensemble selon la revendication 11.

13. Utilisation d'un article selon l'une quelconque des revendications 7 - 10, servant à définir un analyte dans un échantillon.
